# EUROPEAN PATENT SPECIFICATION

(11) **EP 0 672 382 B1**
(45) Date of publication and mention of the grant of the patent: **20.09.2000**
(21) Application number: 95102770.5
(22) Date of filing: 27.02.1995
(51) Int. Cl.: A61B 5/0225

(54) **An electronic blood pressure monitor**
Elektronisches Gerät zur Überwachung des Blutdrucks
Dispositif électronique destiné à la surveillance de la pression sanguine

(30) Priority: 28.02.1994 JP 2875994
(43) Date of publication of application: 20.09.1995
(73) Proprietor: OMRON CORPORATION, Kyoto-shi, Kyoto 616-8025 (JP)
(72) Inventor: Teramoto, Tsutomu, c/o Omron Corp., Nagaokakyo.City, Kyoto 617 (JP)
(74) Representative: WILHELMS, KILIAN & PARTNER Patentanwälte

(56) References cited:
- EP-A- 0 079 306
- EP-A- 0 482 242
- US-A- 4 116 230
- US-A- 4 312 359

## Description

### BACKGROUND OF THE INVENTION

### 1. Field of the Invention

This invention relates to an electronic blood pressure monitor, and more particularly to an improved oscillometric type blood pressure monitor.

### 2. Discussion of the Related Art

There is well known a blood pressure monitor employing an oscillometric method which presses a blood vessel to extract a series of pulse wave components from the blood vessel, for example, by slowly decreasing the pressure in a cuff, computes a pulse wave parameter, for example, a pulse wave amplitude for each pulse wave, stores the pulse wave parameter and the cuff pressure on computation as data, and defines a blood pressure value in view of the obtained cuff-pressure parameter characteristic. Particularly, when a pulse wave amplitude is used for a parameter, the cuff pressure at the higher pressure side when a pulse wave amplitude corresponding to "the maximum pulse wave amplitude X 0.5" is obtained is regarded as the systolic blood pressure and the cuff pressure at the lower pressure side when a pulse wave amplitude corresponding to "the maximum pulse wave amplitude X 0.7" is obtained is regarded as the diastolic blood pressure. In this conventional oscillometric type electronic blood pressure monitor, pulse wave parameters are obtained for each time period by detecting one cycle of a pulse wave, or for each (what is called) window by setting a predetermined section.

The method for recognizing a pulse wave for each time period and computing each pulse wave to obtain parameters such as pulse wave amplitudes and so forth disadvantageously needs a large program capacity or a large storage capacity for recognition and computation. The method for sectioning pulse wave data by a predetermined constant interval to obtain parameters for each section can reduce the size of storage capacity for recognition and data area, but the time interval for section in this method must be designed to be longer than the longest pulse wave time period, considering the persons having a relatively small number of pulses waves. If designed so, any increased number of data cannot be obtained from the person having a relatively large number of pulse waves. In other words, many effective data existing in a patient are shrunk to a small number of data in the conventional monitor.

Blood pressure monitors utilizing the Korotkoff sound technique are known from US-A-A 312 359, FR-A-0 079 306 and US-A-4 116 230. A pressure relief valve is opened periodically for controlled durations, the durations being varied so as to maintain constant increments of cuff pressure decrease. The durations may be changed from a constant time in the beginning to times linked to the pulse rate.

### SUMMARY OF THE INVENTION

It is, therefore, a primary object of this invention to provide an improved electronic blood pressure monitor capable of ensuring the number of data of pulse wave parameters to a some extent without relatively increasing program capacity and storage capacity.

According to the invention there is provided an electric blood pressure monitor as defined in claim 1.

### BRIEF DESCRIPTION OF THE DRAWINGS

Other objectives and advantages of this invention will be more readily apparent from the following detailed description provided in conjunction with the following figures, of which.
Fig. 1 is a schematic block diagram of an electronic blood pressure monitor as a preferred embodiment of this invention;
Fig. 2 is a flow chart showing a whole operation of the monitor of Fig. 1;
Fig. 3 is a detailed flow chart of a window determination process routine in the flow of Fig. 2; and
Fig. 4 is a time chart of pulse wave forms for illustrating the window determination process routine.

### DETAILED DESCRIPTION OF THE INVENTION

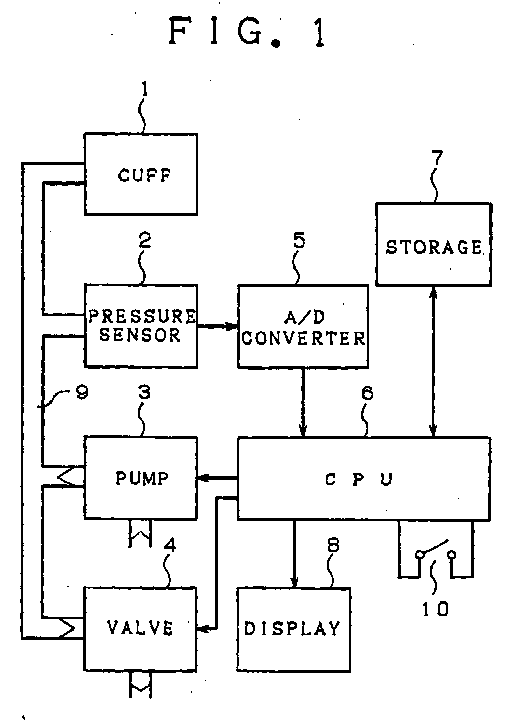

Returning to Fig. 1, there is shown a schematic block diagram showing a construction of an electronic blood pressure monitor as a preferred embodiment of this invention, which includes a cuff 1, a pressure sensor 2, a pump 3, an air exhaustion valve 4, an A/D converter 5, a CPU 6, a storage 7, a display 8, piping 9, and a start switch 10. The cuff 1 is designed to be attached around a patient's arm, but may be designed to be attached to a wrist or a finger of the patient if desired. The pressure sensor 2 is disposed to convert a pressure signal (cuff pressure) into an electric signal. The pump 3 is designed to inflate the cuff 1, and employs an automatic pressurizing pump or a rubber ball for a manual pressurization. The valve 4 is disposed to exhaust the pneumatic system of cuff 1 and piping 9 into the air, and employs an electromagnetic valve for an automatic operation or a manual exhaustion valve for a manual operation. The A/D converter 5 converts the analog electric signal from the pressure sensor 2 into a digital signal.

The CPU 6 includes a function for separating the pressure data read by the A/D converter 5 into a pulse wave component and a static pressure, a function for obtaining pulse wave data such as pulse wave amplitudes from the pulse wave component, a function for computing systolic and diastolic blood pressures based on the obtained pulse wave data and cuff data, a function for controlling pump 3 and valve 4, and a function for switching the width of a window or a section for obtaining pulse wave data.

In this electronic blood pressure monitor, the cuff 1 is inflated to a predetermined pressure value by the pump 3, subsequently deflated at a very slow speed by the valve 4, and systolic and diastolic blood pressures are determined based on cuff pressure data and pulse wave data obtained in the inflating and deflating process. This blood pressure determination method (oscillometric method) itself is well known.

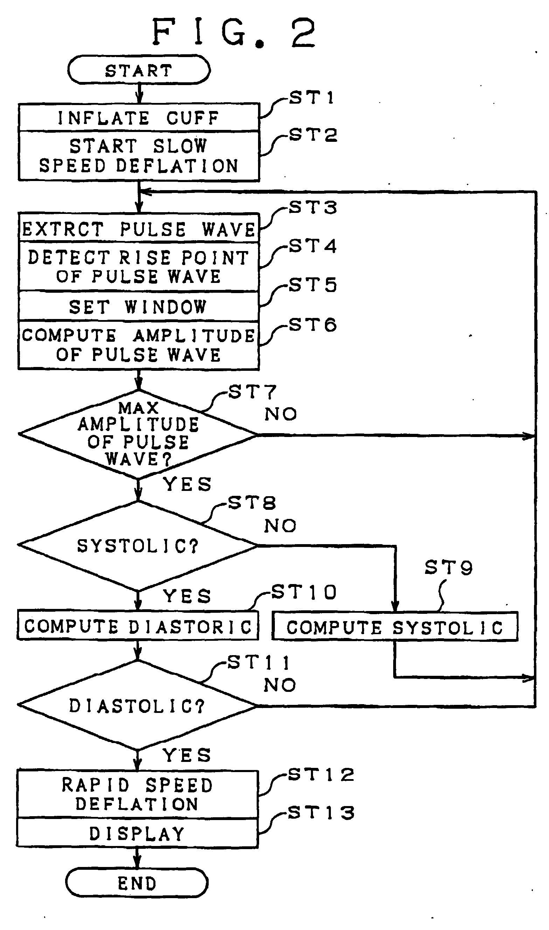

Fig. 2 shows a whole operation of this blood pressure monitor. As the operation starts upon turning on the start switch 10, the pump 3 is turned on to inflate the cuff 1 (step ST1). This pressurization continues until the cuff pressure reaches a preset command or target value. As the cuff 1 is inflated to the target value, the pump 3 is turned off and a very slow speed deflation starts (step ST2), so that the pressure in the cuff 3 is gradually decreased by the valve 4. In this slow speed deflation process, a pulse wave component overlapped on the static pressure component of the cuff pressure is extracted (step ST3), a rise point of a pulse wave is detected (step ST4), a window width is determined in a window determination routine or a set window routine (step ST5), a pulse wave amplitude is computed based on the maximum and the minimum levels within the window width (step ST6), and the pulse wave amplitude and the cuff pressure are stored in the storage 7. The window determination routine will be described later in detail.

As time elapses, the sequence from step ST3 and to step ST7 continues until the maximum pulse wave amplitude is detected (step ST7). As the maximum pulse wave amplitude is detected, the cuff pressure corresponding to the pulse wave amplitude of "the maximum pulse wave amplitude value X 0.5" is determined as the systolic blood pressure. Then the systolic blood pressure is computed (step ST 9).

After computing the systolic blood pressure, the sequences from ST3 to ST8, ST10, and ST11 are repeated to obtain cuff pressure and pulse wave amplitude data. The cuff pressure when a pulse wave amplitude becomes "the maximum pulse wave amplitude X 0.7" is determined as the diastolic blood pressure. Then, diastolic blood pressure is computed (step ST10). After computing the diastolic blood pressure, the valve 4 is widely opened for rapid speed deflation (step ST12), and the systolic and diastolic blood pressures are displayed on the display 8 (step ST13).

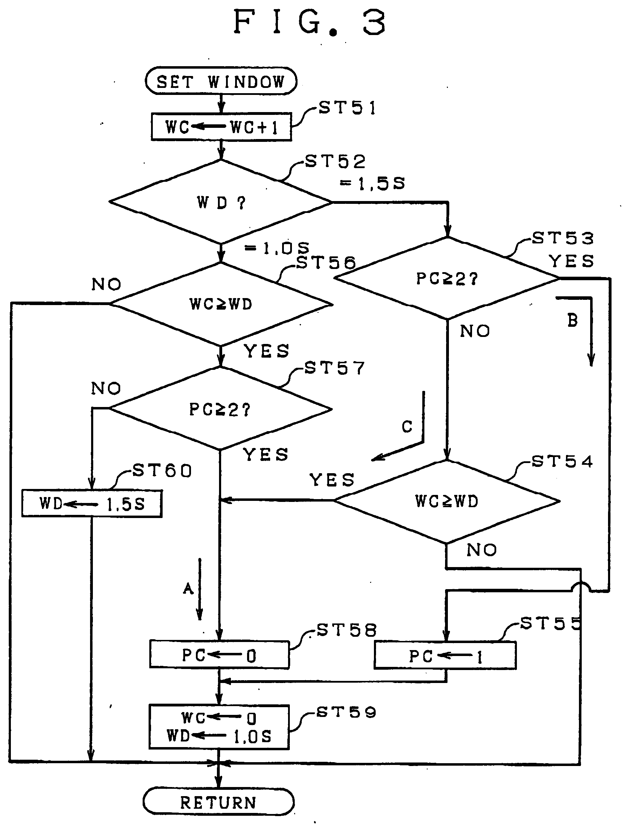

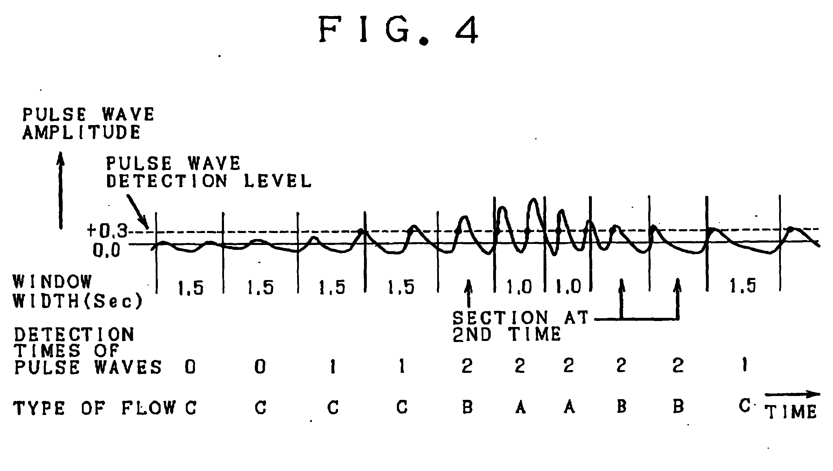

The window determination routine will be described in detail referring to the flow chart of Fig. 3 and the wave form time chart of Fig. 4. A window counter WC is a counter for counting an elapsing time within a window, and increased by "1" each 30 milliseconds. Window data WD represents the width of the determined current window, and is 1.5 seconds or 1.0 seconds. A pulse wave counter PC counts one "1" at a rise point where the pulse wave component level crosses a pulse wave detection level in an increasing direction, and is a counter for counting the number of pulse waves.

Upon entering the window determination routine or set window routine, the window counter WC is increased by one "1" (step ST51), and it is enquired what the window data WD (step STS2) is. At the beginning of slow speed deflation the data is set to a larger side width of 1.5 seconds, and it is enquired if the pulse wave counter PC is two or more (step ST53). As shown is at a left hand side of Fig. 4, the cuff pressure is large at the beginning of low speed deflation and the amplitude of the pulse wave component is small without crossing the pulse wave detection level. Accordingly, a NO response is produced in the step ST53, and it is inquired if the window counter WC count is equal to the window data WD or larger, viz., becomes 1.5 seconds (step ST54). If the sequence is within the window, a NO response is generated and the sequence returns. In this sequence, the window width is not changed, and its subsequent window is also set to 1.5 seconds. In the two windows at the most left hand side, any pulse wave components do not cross the detection level, and its subsequent window is set to 1.5 seconds.

When the amplitude of a pulse wave component becomes large to cross the detection level only once as shown in the third and fourth windows at the left hand side of Fig. 4, the step ST53 produces a NO response and the sequence returns through the step ST54 in the above same sequence. Then, the window width remains 1.5 seconds. As a pulse wave level crosses the pulse wave detection level two times within 1.5 seconds as shown in the fifth window of Fig. 4, viz., the palse wave counter becomes 2, the step ST53 generates a YES response to be applied to the step ST55 to set the pulse wave counter to "1", and the sequence moves to step ST59 where the window counter WC is set to "0", the window data WD is set to 1.0 second, and FWVAL is set to "0" for return. This process allows the sequence to move the subsequent window having a 1.0 second width.

In the subsequent window determination routine, the sequence at the step ST53 where the window data WD is 1.0 seconds moves to step ST56 to inquire whether or not the window counter WC is equal to the window data WD or larger. If the window counter WC is less than 1.0 seconds, the sequence returns. If WC is 1.0 seconds or larger, it is inquired if the pulse counter PC count is 2 or larger (step ST57). If two or more pulse waves are detected as shown in the left hand side sixth window of Fig. 4, the pulse wave counter PC is reset (step ST58), the window counter is set to "0", the window data WD remains to be 1.0 second, and the sequence returns. The window set in this sequence is 1.0 seconds, and the subsequent window is also temporality set to 1.0 seconds. In sixth window from the left of Fig. 4, the window starts at the cross point with the detection level (PC=1 at start), PC=2 is made at the first cross point within the window where the window does not end, and the sequence moves to the subsequent in one second elapsing. The seventh window from the left of Fig. 4 which does not start at the cross point finishes in one second, after pulse wave counting by two detection times of cross point detection (two detection times of pulse waves).

Unless there are two or more times of cross points within one second, a NO response is produced in the step ST57, the window data WD is set to 1.5 seconds (step ST60), and the sequence returns. In the subsequent operation cycle, the step ST52 produces the response of WD=1.5 without detecting the second cross point (step ST53) and the sequence returns until 1.5 seconds elapses (step ST54). If the second cross point is detected before 1.5 seconds elapses, the step ST53 produces YES response and the sequence takes the flow B of steps ST55 and ST59 to finish the window for the next window which corresponds to the eighth and ninth window from the left hand side of Fig. 4.

If 1.5 seconds elapses before detecting the second cross point, the sequence takes the flow C through steps ST53 and ST54 for return where the window finishes upon elapse of 1.5 seconds. Thus, the window width of this embodiment can be switched to either 1.5 or 1.0 seconds. If the second cross point is not detected in one second, the window is sectioned when the second cross point is detected before 1.5 seconds elapse. A width of window is available between the maximum value of 1.5 seconds and the minimum value of 1.0 second.

Thus, according to this invention, a plurality of time widths for sectioning pulse wave data are available and selected corresponding to a pulse wave cycle, whereby the number of effective data can be increased.
(1) The measurement accuracy about a patient having a large number of pulses can be improved.
(2) A sufficient number of data can be obtained in a patient having a large number of pulses even if the deflation speed is high, resulting in reduction of measuring time.
(3) The storage for data area is not necessary to be increased.
(4) The maximum and minimum values of the time width sectioning pulse waves are employed to simplify the recognition of one pulse wave. Wrong recognition can be reduced even about artifact.
(5) The program capacity can be relatively small in comparison with the recognition for each cycle.

While the invention has been described and illustrated with respect to certain embodiments which give satisfactory results, it will be understood by those skilled in the art, after understanding the purpose of the invention, that various other changes and modifications may be made without departing from the scope of the invention, and it is therefore, intended in the appended claims to cover all such changes and modifications.

## Claims

1. An electronic blood pressure monitor, comprising:
a cuff (1) for pressing a blood vessel;
a pressure sensor (2) for detecting the pressure values in said cuff;
extracting means (ST3) for extracting pulse wave components from said blood vessel;
sectioning means (ST5) for sectioning pulse wave components continuously extracted by said extracting means in a pressure changing process of the cuff pressure for sections of a predetermined time period counted by timer means (6);
computing means (ST6) for computing pulse wave parameters from said pulse wave components sectioned by said sectioning means; and
determining means (ST9, ST10) for determining a blood pressure value by selecting a pressure value from the pressure values detected by said pressure sensor, based on the pulse wave parameters computed by said computing means; characterized in that
said monitor further comprises
counting means for counting the number of said pulse wave components in each section sectioned by said sectioning means;
judging means (ST53, ST57) for judging whether or not the number counted by said counting means is a predetermined number; and
section width switching means (ST59, ST60) for shortening the time period of said sections when said counting means counts a predetermined number and lengthening the time period of said sections when said counting means counts less than a predetermined number.

## Patentansprüche

1. Elektronischer Blutdruckmonitor, welcher aufweist:
eine Manschette (1) zum Drücken gegen ein Blutgefäß;
einen Drucksensor (2) zur Feststellung der Druckwerte in der Manschette;
Extrahiermittel (ST3) zum Extrahieren von Pulswellenkomponenten aus dem Blutgefäß;
Unterteilungsmittel (ST5) zum Unterteilen von kontinuierlich mit den Extrahiermitteln in einem Druckänderungsvorgang des Manschettendrucks extrahierten Pulswellenkomponenten in Unterteilungen von einer mit Zeitgebermitteln (6) gezählten bestimmten Zeitdauer;
Berechnungsmittel (ST6) zum Berechnen von Pulswellenparametern aus den mit den Unterteilungsmitteln unterteilten Pulswellenkomponenten; und
Bestimmungsmittel (ST9, ST10) zur Bestimmung eines Blutdruckwertes durch Auswählen eines Druckwerts aus den mit dem Drucksensor festgestellten Druckwerten beruhend auf den mit den Berechnungsmitteln berechneten Pulswellenparametern, dadurch gekennzeichnet, daß
der Monitor ferner
Zählmittel zum Zählen der Anzahl der Pulswellenkomponenten in jeder durch die Unterteilungsmittel unterteilten Unterteilungen;
Beurteilungsmittel (ST53, ST57) zur Beurteilung, ob die mit den Zählmitteln gezählte Anzahl eine bestimmte Anzahl ist; und
Unterteilungsbreitenumschaltmittel (ST59, ST60) zur Verkürzung der Zeitdauer der Unterteilungen, wenn die Zählmittel eine bestimmte Anzahl zählen, und Verlängern der Zeitdauer der Unterteilungen, wenn die Zählmittel weniger als eine bestimmte Anzahl zählen, aufweist.

## Revendications

1. Dispositif électronique de surveillance de la pression sanguine, comprenant :
une manchette (1) pour comprimer un vaisseau sanguin ;
un détecteur de pression (2) pour détecter les valeurs de pressions dans ladite manchette ;
des moyens d'extraction (ST3) pour extraire des composantes d'ondes impulsionnelles desdits vaisseaux sanguins ;
des moyens de sectionnement (ST5) pour sectionner des composantes d'ondes impulsionnelles extraites en continu par lesdits moyens d'extraction dans un processus de modification de pression de la pression de la manchette pour des sections d'une période de temps prédéterminée comptée par des moyens de comptage de temps (6) ;
des moyens de calcul (ST6) pour calculer des paramètres d'ondes impulsionnelles à partir desdites composantes d'ondes impulsionnelles sectionnées par lesdits moyens de sectionnement ; et
des moyens de détermination (ST9, ST10) pour déterminer une valeur de pression sanguine par sélection d'une valeur de pression à partir des valeurs de pressions détectées par ledit détecteur de pression, en se basant sur les paramètres d'ondes impulsionnelles calculés par lesdits moyens de calcul ; caractérisé en ce que
ledit dispositif de surveillance comprend en outre
des moyens de comptage pour compter le nombre desdites composantes d'ondes impulsionnelles dans chaque section, sectionnées par lesdits moyens de sectionnement ;
des moyens d'estimation (ST53, ST57) pour estimer si le nombre compté par lesdits moyens de comptage est ou non un nombre prédéterminé ; et
des moyens de commutation de largeur de section (ST59, ST60) pour raccourcir la période de temps desdites sections lorsque lesdits moyens de comptage comptent un nombre prédéterminé et allonger la période de temps desdites sections lorsque lesdits moyens de comptage comptent un nombre intérieur à un nombre prédéterminé.
